# EUROPEAN PATENT APPLICATION

(11) **EP 4 651 151 A1**
(43) Date of publication of application: **19.11.2025**
(21) Application number: 24176807.6
(22) Date of filing: 17.05.2024
(51) Int. Cl.: G16H 80/00, G16H 40/20, G06Q 10/1093, G16H 10/00

(54) **METHOD OF CONDUCTING CONSULTATIONS, IN PARTICULAR MEDICAL ONES, BETWEEN MULTIPLE USERS, A COMPUTER PROGRAM AND A COMPUTER-READABLE DATA CARRIER**

(71) Applicant: Holysz, Karolina, 04-041 Warszawa (PL)
(72) Inventor: Holysz, Karolina, 04-041 Warszawa (PL)
(74) Representative: AOMB Polska Sp. z.o.o.

(57) **Abstract**

The invention provides a method for communication between users, in particular for conducting medical consultations. The method is carried out on a server and client devices. The invention also provides a computer program and a computer-readable data carrier, comprising instructions which when executed by a computer cause the computer carry out the steps of conducting consultations. A method for conducting consultations, in particular medical ones, between multiple users, wherein the method is carried out on a server and the method comprises the steps of:
a) initiation of a topic thread on a server by the first user, which topic thread may be displayed to at least one user on a client device,
b) receiving, by the server, of information about the need for an additional user in the topic thread, the additional user meeting all of at least one requirement,
c) searching, by the server, for an additional user from a set of users who are presently available,
d) sending, preferably by the server, of an invitation to at least one user who meets all of at least one requirement,
e) upon acceptance of the invitation, joining the user to said topic thread.

## Description

### Technical Field

The invention provides a method for communication between users, in particular for conducting medical consultations. The method is carried out on a server and on client devices. The invention also provides a computer program and a computer-readable data carrier comprising instructions which when executed by a computer cause the computer to carry out the steps of the method of conducting consultations.

### Prior Art

Document CN102496133A discloses a doctor-patient communication terminal. The doctor-patient communication terminal is a set of dedicated tools for immediate communication between a hospital and a patient, and it is comprised of nurse working stations, doctor-patient intelligent touchscreens, novel mobile phone platforms, table-top personal computer platforms, doctor working stations, a terminal for collecting information concerning patient rooms, wireless call systems, cloud television devices, etc. The invention is aimed at improving doctor-patient information, modification of relations of doctors and patients, and help for doctors and patients in deep communication, immediate follow-up consultations for the patients, and providing consultation comfort for patients.

Document US20130073310A1 discloses an interface for interactions between patients, medical professionals and third parties. Patients, doctors and third parties may render information concerning medical procedures, products and services accessible by means of computer and internet human-machine interfaces which implement a highly interactive and personalized mobile health interface. Third parties may use the system for promotion of products and services by means of targeted advertisement and promotion. Medical professionals may track marketing activities of third parties in order to integrate the services and products of such third parties with the patient care.

It is the object of the invention to provide a platform for virtual real-time communication between users, in particular doctors with particular areas of specialization, and other medical industry representatives. The solution is intended to enhance communication, in particular when for a specific topic thread specialised information is required or the attention of a user who meets all of one or more specific conditions.

### Subject-Matter of the Invention

The invention provides a method for conducting consultations, in particular medical ones, between multiple users, wherein the method is embodied on a server, and the method comprises the steps of:
a) initiating a topic thread on a server by the first user, which topic thread may be displayed to at least one user on a client device,
b) receiving, by the server, of information about the need for an additional user in the topic thread, the additional user meeting all of at least one requirement,
c) searching, by the server, for an additional user from a set of users who are presently available,
d) sending, preferably by the server, of an invitation to at least one user who meets all of at least one requirement,
e) upon acceptance of the invitation, joining the user to the topic thread.

Preferably, at least one of the users is a verified user.

In this embodiment, during registration of a new user, the method comprises step a0) of registration and verification of a user, and preferably verification of entitlements such as the authority to practice the respective profession.

Topic thread has a private or public nature, and the public nature means that it is visible and active for all the users, while the private nature means that it is visible and active for some selected users.

In a preferable embodiment, in step a), of during initiation of a topic thread at least one requirement is indicated, in particular concerning an appropriate medicine specialization, medicament-related consultations or consultations concerning a specific medical case of a patient.

Preferably, information about the need for an additional user, with the at least one requirement, is provided to the server manually by the first user.

Information about the need for an additional user is filtered with regard to the age, experience, establishment of the profession being practised, academic degree, country where the profession is practiced, or language.

In this embodiment, server obtains information about the need for an additional user, preferably with at least one requirement, by means of the analysis of the contents in the topic thread, preferably by means of the analysis of predefined key words or with the use of artificial intelligence.

In a preferable embodiment, a topic thread is initiated by the first user, who also has special entitlements, and upon communicating the need for an additional user in the topic thread, the server, based on the available appointment times defined by each of the users without special entitlements, sends an invitation for at least one appointment time to at least one user without special entitlements, and preferably the at least one user without special entitlements has to accept at least one appointment time.

The user, in reply to the invitation related to the communicated need for an additional user, may either accept the invitation and become the additional user for the specific topic thread, or send feedback to inform on rejection of the invitation or on availability of said user.

Preferably, the method comprises step a1) of enclosing at least one file to the topic thread, and preferably at least one file comprises medical data such as results of tests, and more preferably, during enclosing is anonymized by the server or the client device.

In this embodiment, the user may, at any stage, download results of patient's tests from the server, which data preferably are passed through a patient personal data censoring module.

Topic thread is accessible for a preset duration time, and the preset duration time is defined by the user and/or is predefined, preferably the preset duration time lasts 24-72 hours.

Another object of the invention is a computer program comprising instructions which when the program is executed by a computer cause the computer carry out the method steps.

A third object of the invention is a computer-readable data carrier comprising instructions which when executed by a computer cause the computer carries out the program steps.

### Advantageous Effects of the Invention

The solution according to the invention enhances effectiveness of information exchange between the users. In a structured manner, the user's attention is directed towards issues that require attention of the user who meets specific criteria. The invention also provides, in a preferable embodiment, functionalities which provide a possibility to discuss topics that require anonymization of data, e.g., patient data which may be included in test results.

### Detailed Description of Embodiments of the Invention

The object of the invention is to provide efficient communication between users. For the sake of simplicity, the invention will be presented in an example in the medical industry, by this by no means should be construed as limitation to this industry only. The subject method enables exchange of information by numerous users in real time. Users may communicate to each other in relation to topics of a given field 24 hours a day and they may expect a quick response.

Method for conducting consultations between multiple users is implemented on a server accessible for users via client devices. The method comprises, in a most general implementation, steps a-d. Step a) consists on initiation of a topic thread on a server by the first user, which topic thread may be displayed to at least one user on a client device. Step b) consists on receiving, by the server, of information about the need for an additional user in the topic thread, the additional user meeting all of at least one requirement. Step c) consists on searching, by the server, for an additional user from a set of users who are presently available. Step d) consists on sending, preferably by the server, of an invitation to at least one user who meets all of at least one requirement In step e), upon acceptance of the invitation, the user is joined to the topic thread.

Client device, for the purposes of this application, should be broadly understood as a computer, smartphone, smartwatch or some other device which enables sending and transferring information from and to the server.

Throughout this application a term user is applied which should be understood as any person who has access to the server with the use of the method according to the invention. The first user is the user who initiated the topic thread and is the owner, moderator or administrator thereof. The term additional user should be understood as a user who meets criteria for a given specific topic thread and his/her presence is advisable for more efficient resolving of the issue. Such criteria may relate to, e.g., knowledge, entitlements or other competence, qualification or skill, but also the equipment at hand or experience. Such criteria should be evaluated broadly within the context of a specific industry or field in which the server implementing the solution according to the invention operates.

The term topic thread also should be understood broadly as a defined, within the server, space in which in a structured manner exchange of information is possible. The form in which the information is exchanged is any arbitrary form. Most often this will be in the form of text, audio or video. Topic thread may be also understood as an ad-hoc arranged or planned teleconference. It cannot be excluded for the server to provide more than one form of communication between the users. For example, a thread may have in its basic form a text form and additionally remind about the thread at a forum or conversation in an application for messages, and if the users, in particular the first user, recognize that a conversation in a form of a teleconference of a voice call is necessary, then the server also may enable such conversation.

Within step c) it is indicated that the server searches for an additional user from a set of users who are presently available. A situation may be conceived where the user indicated criteria are not met by any one of the available users or the user has a preference for the presence of specific users who are at that time unavailable - in this situation it is not possible to indicate available users. In urgent cases it is preferable for the additional user to be joined to the thread possibly promptly and this makes the response time shorter. It should be noted that fast joining may imply a necessity to limit the conditions or joining several additional users who jointly meet all the requirements.

In d) it is indicated that the invitation is sent by the server to another user. This is the most obvious solution, and it cannot be excluded for the invitation to be sent as a link to a topic thread in a form of an email message, sms or in another manner with the use of a client device, without intermediaries of the server.

Topic threads may be, for example, divided into medical specializations such as surgery, neurology, etc. It is also possible to divide the topic threads with based on the country or language used for exchange of information. Users may search for interesting topic threads or initiate new ones. Such ordering facilitates searching appropriate topics within short times.

In a preferable embodiment, at least one of the users is a verified user. This means that his/her data and information provided are verified - this may be effected by means of communication between the server and data bases that enable identity verification (for example, EPUAP or electronic banking), entitlements (lists of persons with medical, lawyer entitlements held by the ministries or chambers), trainings (databases of academies or schools), as well as by obtaining information directly from the employers (hospitals, universities).

In one embodiment, during registration of a new user, the method according to the invention comprises step a0) of registering and verifying the user's identity, and preferably entitlements such as professional entitlements.

In a specific embodiment, topic thread has a private or public nature, wherein the public nature means that it is visible and active for all the users, while the private nature means that it is visible and active for some selected users. This is applicable in particular in the case where the first user is not wishing for his/her inquiry to be publicly known, and he/she would like as few persons as possible to know about a given issue. The solution in a form of a private topic thread enables enhancing of the security level for data accessibility by means of limitation of the potential contents recipients.

In a further embodiment, in step a), of during initiation of a topic thread at least one requirement is indicated. These requirements concern in particular an appropriate medicine specialization, medicament-related consultations or consultations concerning a specific medical case of a patient.

In another embodiment, information about the need for an additional user, preferably with at least one requirement, is provided to the system manually by the first user. This solution is simple for implementation and provides the first user with complete control as to who is invited. For example, information about the need for an additional user is filtered based on the age, experience, establishment of the profession being practised, academic degree, country where the profession is practiced, or language.

In another, non-excluded example, the server obtains information about the need for an additional user, preferably with at least one requirement, by means of the analysis of the contents in the topic thread. Analysis of the contents may be effected, for example, by means of the analysis of predefined key words or with the use of artificial intelligence. In the case of automatic suggestions for specific users or requirements, the user, in particular the first user, may autonomously change the proposed criteria or accept them. In another implementation, it may be assumed that the user solely indicates the need and the server autonomously selects conditions and additional user without further communications. Suitably to the implementation, information about the need for joining of an additional user may be generated by the first user only, while in other implementations, by some selected users, such as additional users, or even by any user.

In another embodiment, topic thread is initiated by the first user who also has special entitlements, and upon communicating the need for an additional user in the topic thread the server, based on the available appointment times defined by each of the users without special entitlements, sends an invitation for at least one appointment time to at least one user without special entitlements. Preferably, a user without special entitlements has to accept at least one appointment time. A user with special entitlements may be, for example, a user who pays for such entitlements or for some other reason is privileged. An exemplary user with special entitlements may be a sales representative who is given an opportunity to present, e.g., medicaments to doctors who may thus obtain knowledge about specific products to be used for future prescriptions for the patients.

In a further embodiment, the user, in reply to the invitation related to the communicated need for an additional user, may either accept the invitation and become the additional user for a specific topic thread, or send feedback to inform on rejection of the invitation or on availability of said user.

Additionally it should be noted, that the server may collect data concerning typical availability of the user. For example, based on history data, the server may store information that a given user is most often available on Mondays between 11 to 17 o'clock and on Thursdays between 8 to 12 o'clock. Such information may be used as prompts to indicate convenient appointment times or for other users who are willing to contact a given user.

In an embodiment, the method comprises step a1) of enclosing at least one file to the topic thread. The file may, for example, comprise medical data such as test results. In a most preferable implementation, during enclosing, the file is anonymized by the server or by the client device. In a similar manner, the user may, at any stage, download results of patient's tests from the server, which data preferably pass through a patient personal data censoring module. This ensures that all the necessary information are conveniently accessible for the users, in particular, additional users.

In still another embodiment, topic thread is accessible for a preset duration time, and the preset duration time is defined by the user and/or is predefined, preferably the preset duration time lasts 24-72 hours. This solution enhances anonymity by providing temporary access to data which may be sensitive data, and reduces the disk space required for the server. Regardless of the above, topic threads may be additionally labelled as urgent, atypical, interesting, etc.

Other aspects of the invention are a computer program comprising instructions which when executed by a computer cause the computer carry out the steps of the method defined above, as well as a computer-readable data carrier comprising instructions which when executed by a computer cause the computer carry out the steps of the method described above.

One example of the server carrying out the method according to the invention is a platform for doctors and medicine professionals. On such a server, a doctor who needs to consult on the case, may initiate a separate topic thread. This thread may be visible for all, but as well it may be accessible solely for other users selected by the first user. In such a thread, the server detects which other specialist is necessary to analyse thoroughly the case and searches for users to meet specific criteria. Such criteria may also originate from the first user who may, for example, indicate that he/she needs to consult a doctor, an oncologist. Then the system selects, suitably to possible further criteria, such as e.g. knowledge of the Polish language, available users and assigns one of them to the thread by communicating the need for consultation. Upon acceptance, information is exchanged in order to make a diagnosis. To the topic thread, files such as images or texts with test results may be enclosed.

In the operation of the platform, it may result that interaction with a specific user is desired. In such case, the service may inform on current availability of the user (provided that he/she meets all the requirements for a specific topic thread). If the specific user is not available, the server may, based on the logging history, indicate when next logging-in of the user is expected to occur and this will enable a decision whether it is possible to wait for such next logging-in or not.

If urgent consultation is needed, the user may resign from some of his criteria (e.g., knowledge of the Polish language becomes changed into knowledge of the English language) and then any doctor from any country, available at that time, may receive a notification. In a situation where consultation is urgently needed, e.g., at 5 o'clock in the morning, it may turn out that in a given country there are few specialists available, while in another part of the world there are numerous users available for whom this time falls in the middle of the day.

Within the context of the server, it is possible to imagine users with special entitlements - these may be, for example, sales representatives of pharmaceutical companies who would like to present their products to the doctors. The server may require each user to conduct a specific number of consultations - and this may be a condition for access to the service. In such case the users show their availability and the sales representative, being a user with special entitlements, suggests meetings and has a possibility to offer new solutions useful in everyday work.

## Claims

1. A method for conducting consultations, in particular medical ones, between multiple users, wherein the method is carried out on a server and the method includes the steps of:
a) initiation of a topic thread on a server by the first user, which topic thread may be displayed to at least one user on a client device,
b) receiving, by the server, of information about the need for an additional user in said topic thread, the additional user meeting all of at least one requirement,
c) searching, by the server, for an additional user from a set of users who are presently available,
d) sending, preferably by the server, of an invitation to at least one user who meets all of said at least one requirement,
e) upon acceptance of said invitation, joining said user to said topic thread.

2. The method for conducting consultations according to claim 1, **wherein** at least one of said users is a verified user.

3. The method for conducting consultations according to claims 1 or 2, **wherein,** during registration of a new user, the method comprises step a0) of registering and verification of user identity, and preferably also of entitlements such as the authority to practice the profession.

4. The method for conducting consultations according to any one of claims 1-3, **wherein** said topic thread has a private or public nature, wherein said public nature means that it is visible and active for all said users, while said private nature means that it is visible and active for some selected users.

5. The method for conducting consultations according to any one of claims 1-4, **wherein,** in step a), of during initiation of said topic thread, at least one requirement is indicated, in particular concerning an appropriate medicine specialization, medicament-related consultations or consultations on a specific medical case of a patient.

6. The method for conducting consultations according to any one of claims 1-5, **wherein** said information about the need for an additional user, preferably with at least one requirement, is provided to the server manually by the first user.

7. The method for conducting consultations according to claim 6, **wherein** said information about the need for an additional user is filtered based on the age, experience, establishment of the profession being practised, academic degree, country where the profession is practiced, or language.

8. The method for conducting consultations according to any one of claims 1-7, **wherein** said server obtains said information about the need for an additional user, preferably with at least one requirement, by means of the analysis of the contents in the topic thread, preferably by means of the analysis of predefined key words or with the use of artificial intelligence.

9. The method for conducting consultations according to any one of claims 1-8, **wherein** said topic thread is initiated by the first user who also has special entitlements, and upon communicating the need for an additional user in a topic thread, the server, based on the available appointment times defined by each of the users without special entitlements, sends an invitation for at least one appointment time to at least one user without special entitlements, and preferably the at least one user without special entitlements has to accept at least one appointment time.

10. The method for conducting consultations according to any one of claims 6-8, **wherein** said user, in reply to said invitation related to said communicated need for an additional user, may either accept said invitation and become an additional user for said specific topic thread, or send feedback to inform on rejection of said invitation or on availability of said user.

11. The method for conducting consultations according to any one of claims 1-10, **wherein** it comprises a step a1) of enclosing at least one file to said topic thread, and preferably at least one file comprises medical data such as results of tests, and more preferably, during enclosing it is anonymized by the server or client device.

12. The method for conducting consultations according to any one of claims 1-11, **wherein** said user may, at any stage, download results of the patient's tests from the server, and such data are preferably passed through a patient personal data censoring module.

13. The method for conducting consultations according to any one of claims 1-12, **wherein** said topic thread is accessible for a preset duration time and said preset duration time is defined by the user and/or is predefined, preferably said preset duration time lasts 24-72 hours.

14. A computer program comprising instructions which when executing said program by a computer cause said computer carry out the steps of the method according to any one of claims 1-13.

15. A computer-readable data carrier comprising instructions which upon execution by a computer cause said computer to carry out the steps of the method according to any one of claims 1-13.
